# EUROPEAN PATENT APPLICATION

(11) **EP 4 685 509 A1**
(43) Date of publication of application: **28.01.2026**
(21) Application number: 25191226.7
(22) Date of filing: 23.07.2025
(51) Int. Cl.: G01R 33/54

(54) **MEDICAL IMAGING SUPPORT DEVICE, METHOD FOR OPERATING MEDICAL IMAGING SUPPORT DEVICE, PROGRAM, AND MEDICAL IMAGING APPARATUS**

(30) Priority: 26.07.2024 JP 2024121348
(71) Applicant: FUJIFILM Corporation, Tokyo 106-8620 (JP)
(72) Inventor: UCHIO, Yoshiki, Tokyo, 1068620 (JP); KITAMOTO, Shizue, Tokyo, 1068620 (JP); HAMADA, Kota, Tokyo, 1068620 (JP)
(74) Representative: Dehns Germany Partnerschaft mbB

(57) **Abstract**

Provided are a medical imaging support device, a method for operating a medical imaging support device, a program, and a medical imaging apparatus that achieve accurate and rapid recognition of a body position of a subject. A medical imaging support device includes a second memory that stores a correspondence relationship between a type of a receive coil and a plurality of steps of determining a body position of a subject, acquires receive coil information indicating a type of a receive coil used to capture a medical image of the subject, determine whether or not the plurality of steps need to be performed according to the receive coil information, and perform a step determined to need to be performed.

## Description

### BACKGROUND OF THE INVENTION

### 1. Field of the Invention

The present disclosure relates to a medical imaging support device, a method for operating a medical imaging support device, a program, and a medical imaging apparatus.

### 2. Description of the Related Art

In a medical imaging apparatus, such as an MRI apparatus, a technician sets in advance an imaging condition such as a body position of a subject. The subject lies on a bed in a posture corresponding to the set condition. The bed on which the subject lies is inserted into an imaging space with an orientation corresponding to the set condition, and the imaging of the subject is started.

In the capture of a medical image, a body position of the subject different from the preset body position may be intentionally applied. In addition, in the capture of the medical image, the body position of the subject different from the preset body position may be applied by mistake. The body position of the subject different from the preset body position may cause reimaging.

JP2022-88166A discloses a medical information processing apparatus that performs image recognition on a captured image of a subject obtained by an optical imaging system to recognize a body position of the subject including the direction in which the subject is inserted into the gantry.

### SUMMARY OF THE INVENTION

However, in a case where the medical image is captured using the MRI apparatus, a part of the body of the subject is often hidden due to the attachment of a receive coil, covering with a blanket, and the like, and there is a concern that only the image recognition based on the captured image of the subject will be insufficient to recognize the subject. At least one of the use of a computer having relatively high processing performance or the securing of a relatively long processing time is required in order to achieve high-precision image recognition.

The apparatus disclosed in JP2022-88166A has the above-described problem of the image recognition of the subject, but JP2022-88166A does not specifically disclose a solution to the above-described problem.

The present disclosure has been made in view of these circumstances, and an object of the present disclosure is to provide a medical imaging support device, a method for operating a medical imaging support device, a program, and a medical imaging apparatus that achieve accurate and rapid recognition of a body position of a subject.

According to a first aspect of the present disclosure, there is provided a medical imaging support device comprising: a processor; a first memory that stores a program to be executed by the processor; and a second memory that stores a correspondence relationship between a type of a receive coil and a plurality of steps of determining a body position of a subject. The processor is configured to: acquire receive coil information indicating a type of a receive coil used to capture a medical image of the subject; determine whether or not the plurality of steps need to be performed according to the receive coil information; and perform a step determined to need to be performed.

According to the medical imaging support device of the first aspect, a step of determining the body position of the subject is determined according to the receive coil information. Therefore, the accurate and rapid recognition of the body position of the subject is achieved according to the receive coil used. In addition, high-precision image recognition is not required, and an unnecessary operation of other devices is suppressed.

The same hardware may be applied to the first memory and the second memory, or different hardware components may be applied thereto.

The body position of the subject includes the posture of the subject and the orientation of the subject with respect to a traveling direction of the bed.

The medical image may be a reconstructed image reconstructed from imaging data. The reconstructed image may be a two-dimensional image or a three-dimensional image.

According to a second aspect, in the medical imaging support device according to the first aspect, the second memory may store, as the plurality of steps, a first step of determining the body position of the subject according to the type of the receive coil and a second step of determining the body position of the subject according to a connection position of the receive coil in a bed, and the processor may be configured to perform the second step in a case where the body position of the subject is not determined in the first step.

According to a third aspect, in the medical imaging support device according to the second aspect, the processor may be configured to: acquire receive coil connection position information indicating to which of a plurality of second terminals disposed at different positions of the bed a first terminal provided in an electric wire of the receive coil is connected; and perform the second step using the receive coil connection position information.

According to a fourth aspect, in the medical imaging support device according to the second aspect or the third aspect, the second memory may store, as the plurality of steps, a third step of determining the body position of the subject based on at least one of subject feature information indicating a feature of the subject or receive coil feature information indicating a structural feature of the receive coil attached to the subject, and the processor may be configured to: acquire at least one of the subject feature information or the receive coil feature information; and perform the third step in a case where the body position of the subject is not determined in the second step.

According to a fifth aspect, in the medical imaging support device according to the fourth aspect, the subject feature information may include a physical feature of the subject obtained by analyzing a captured image of the subject.

According to a sixth aspect, in the medical imaging support device according to the fourth aspect, the subject feature information may include a result of face recognition on a captured image of the subject.

According to a seventh aspect, in the medical imaging support device according to the sixth aspect, the processor may be configured to: determine a posture of the subject placed on the bed based on the result of the face recognition in the third step.

According to an eighth aspect, in the medical imaging support device according to any one of the fourth to seventh aspects, the receive coil feature information may be generated based on an analysis result of a captured image of the receive coil attached to the subject.

According to a ninth aspect, in the medical imaging support device according to any one of the first to eighth aspects, the processor may be configured to perform update of the correspondence relationship to add a type of a receive coil that is not stored in the second memory.

According to a tenth aspect, in the medical imaging support device according to any one of the first to ninth aspects, the processor may be configured to update the correspondence relationship based on information of a step of determining the body position of the subject used in a facility in which a medical imaging apparatus is provided.

According to an eleventh aspect, in the medical imaging support device according to any one of the first to tenth aspects, the second memory may store the correspondence relationship for each operator, and the processor may be configured to: acquire operator information; and refer to the correspondence relationship based on the operator information.

According to a twelfth aspect of the present disclosure, there is provided a method for operating a medical imaging support device. The method is executed by a computer that includes a second memory storing a correspondence relationship between a type of a receive coil and a plurality of steps of determining a body position of a subject and that functions as the medical imaging support device. The method comprises: acquiring receive coil information indicating a type of a receive coil used to capture a medical image of the subject; determining whether or not the plurality of steps need to be performed according to the receive coil information; and performing a step determined to need to be performed.

According to the method for operating a medical imaging support device of the twelfth aspect of the present disclosure, it is possible to obtain the same operation and effect as those of the medical imaging support device according to the first aspect. The configuration requirements of the medical imaging support device according to the second to eleventh aspects can be applied as configuration requirements of the method for operating a medical imaging support device according to other aspects.

According to a thirteenth aspect of the present disclosure, there is provided a program causing a computer that includes a second memory storing a correspondence relationship between a type of a receive coil and a plurality of steps of determining a body position of a subject and that functions as a medical imaging support device to implement: a function of acquiring receive coil information indicating a type of a receive coil used to capture a medical image of the subject; a function of determining whether or not the plurality of steps need to be performed according to the receive coil information; and a function of performing a step determined to need to be performed.

According to the program of the thirteenth aspect of the present disclosure, it is possible to obtain the same operation and effect as those of the medical imaging support device according to the first aspect. The configuration requirements of the medical imaging support device according to the second to eleventh aspects can be applied as configuration requirements of the program according to other aspects.

According to a fourteenth aspect of the present disclosure, there is provided a medical imaging apparatus comprising: an imaging unit that images a subject and generates imaging data of the subject; an image reconstruction unit that generates a reconstruction based on the imaging data; a processor; a first memory that stores a program to be executed by the processor; and a second memory that stores a correspondence relationship between a type of a receive coil and a plurality of steps of determining a body position of the subject. The processor is configured to: acquire receive coil information indicating a type of a receive coil used to capture a medical image of the subject; determine whether or not the plurality of steps need to be performed according to the receive coil information; and perform a step determined to need to be performed.

According to the medical imaging apparatus according to the fourteenth aspect of the present disclosure, it is possible to obtain the same operation and effect as those of the medical imaging support device according to the first aspect. The configuration requirements of the medical imaging support device according to the second to eleventh aspects can be applied as configuration requirements of the medical image capturing apparatus according to other aspects.

According to the present disclosure, the step of determining the body position of the subject is determined according to the receive coil information. Therefore, the accurate and rapid recognition of the body position of the subject is achieved according to the receive coil used.

### BRIEF DESCRIPTION OF THE DRAWINGS

Fig. 1 is a perspective view showing an appearance of an exemplary magnetic resonance imaging apparatus.
Fig. 2 is a diagram showing a schematic configuration of an inside of the MRI apparatus.
Fig. 3 is a block diagram showing an example of a hardware configuration of an information processing apparatus used in the MRI apparatus.
Fig. 4 is a flowchart showing an imaging support method according to an embodiment.
Fig. 5 is a table showing a correspondence relationship between a type of a receive coil and a step of determining a body position of a subject.
Fig. 6 is a schematic view showing the subject for which a neuro coil is used.
Fig. 7 is a schematic view showing the subject for which a foot ankle coil is used.
Fig. 8 is a schematic view showing the subject for which a torso coil is used.
Fig. 9 is a schematic view showing the subject for which a blanket is used.
Fig. 10 is a schematic view showing the subject for which a breast coil is used.
Fig. 11 is a schematic view showing the subject in a case where both eyes are recognized in face recognition of the subject.
Fig. 12 is a schematic view showing the subject in a case where one eye is recognized in the face recognition of the subject.
Fig. 13 is a functional block diagram showing an electric configuration of an imaging support device according to the embodiment.

### DESCRIPTION OF THE PREFERRED EMBODIMENTS

Hereinafter, preferred embodiments of the present invention will be described in detail with reference to the accompanying drawings. In addition, in the following description and the accompanying drawings, the same components are denoted by the same reference numerals, and a redundant description thereof will be omitted. Further, in the following embodiments, in a case where a plurality of components are described and listed, it can be interpreted that at least one of the plurality of components is included.

### Overview of MRI Apparatus

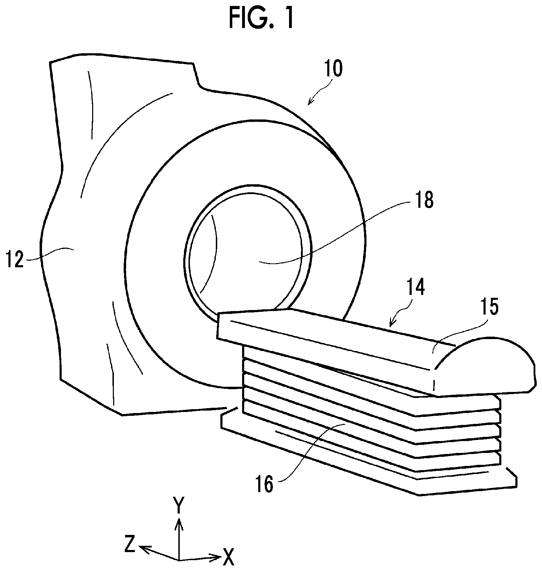

Fig. 1 is a perspective view showing an appearance of an exemplary magnetic resonance imaging apparatus. An MRI apparatus 10 comprises a gantry 12, which is an imaging apparatus main body, and a bed 14. The gantry 12 includes a cylindrical imaging space 18 that is called a bore. In the gantry 12, a static magnetic field generating magnet, a gradient magnetic field coil, and various other coils for generating a magnetic field in the imaging space 18 are disposed. In addition, MRI is an abbreviation of Magnetic Resonance Imaging.

The bed 14 comprises a top plate 15 and a leg portion 16 that supports the top plate 15. The bed 14 is disposed at a position on a front side of the gantry 12. The top plate 15 can be advanced into the imaging space 18 and can be retreated from the imaging space 18 by a drive mechanism (not shown) provided in the leg portion 16. The bed 14 may be configured to be fixed to the gantry 12 or may be a mobile dockable bed that is attachable to and detachable from the gantry 12.

In the MRI apparatus 10, three axes of the apparatus, which are three-dimensional orthogonal coordinate axes of a real space including the imaging space 18, are defined. A direction of each of the three axes of the apparatus is uniquely determined from the gantry 12 and the bed 14. A Z direction in Fig. 1 is a static magnetic field direction, a Y direction is a vertical direction, and an X direction is a direction orthogonal to the Y direction and the Z direction.

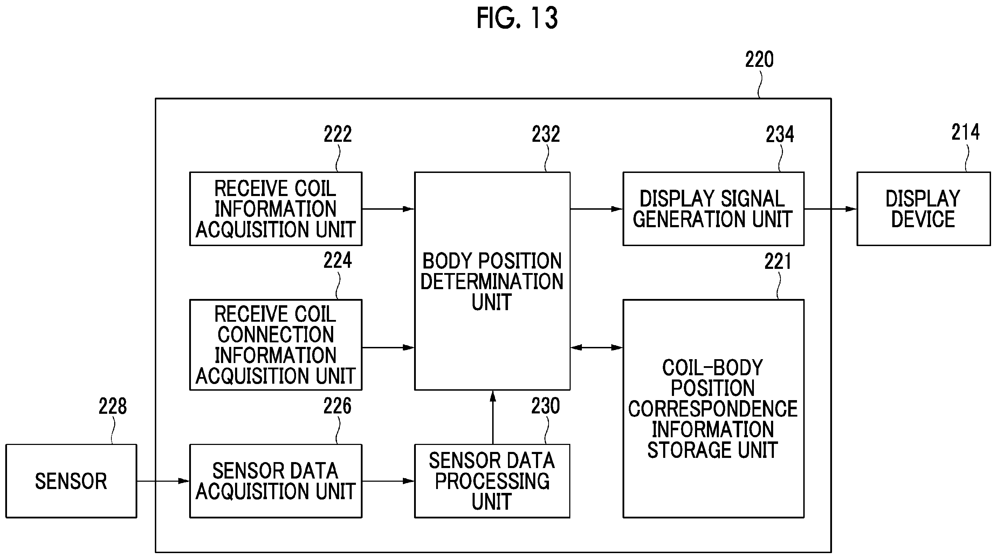

The MRI apparatus 10 comprises a sensor that detects a subject placed on the bed 14, which is not shown in Fig. 1. The sensor is disposed at a position where the sensor can detect the whole body of the subject, such as on a surface of the gantry 12 closer to the bed 14 or on a ceiling of an examination room in which the MRI apparatus 10 is disposed. The sensor may be an optical camera that images the whole body of the subject. The sensor is denoted by reference numeral 228 and is shown in Fig. 13.

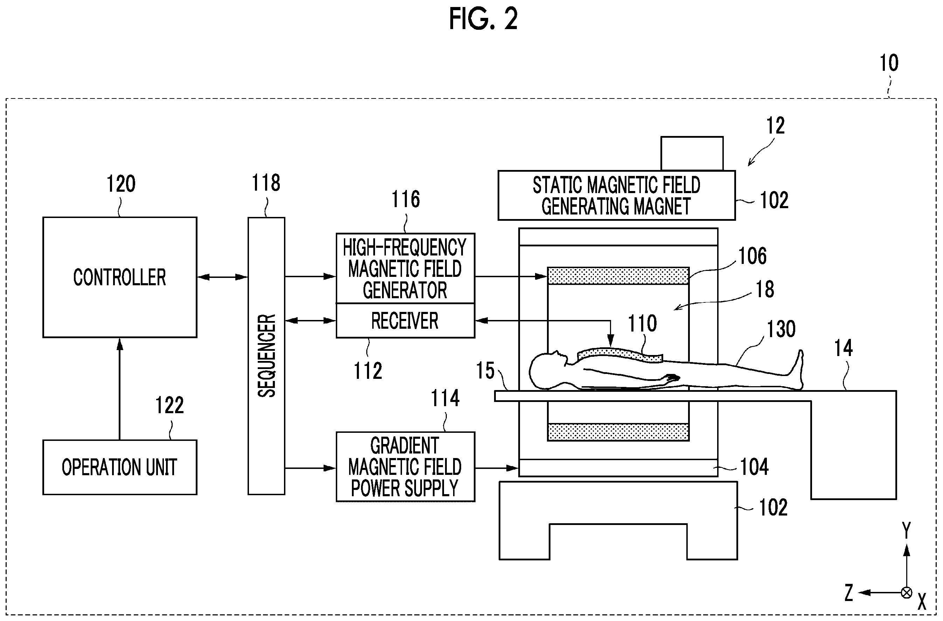

Fig. 2 is a diagram showing a schematic configuration of an inside of the MRI apparatus 10. The MRI apparatus 10 comprises a static magnetic field generating magnet 102, a gradient magnetic field coil 104, an RF transmission coil 106, a receive coil 110, a receiver 112, a gradient magnetic field power supply 114, and a high-frequency magnetic field generator 116. In addition, RF is an abbreviation of Radio Frequency.

The static magnetic field generating magnet 102 generates a uniform static magnetic field in the imaging space 18. The static magnetic field generating magnet 102 includes a static magnetic field generation source of a permanent magnet type, a normal conducting type, or a superconducting type. The gradient magnetic field coil 104 generates a gradient magnetic field in the imaging space 18. The gradient magnetic field coil 104 is composed of gradient magnetic field coils in directions of three axes of the X-axis, the Y-axis, and the Z-axis which are a real space coordinate system. The real space coordinate system can be referred to as a stationary coordinate system. Each gradient magnetic field coil is connected to the gradient magnetic field power supply 114 and is supplied with a current. Therefore, gradient magnetic fields are generated in the directions of three axes of the X-axis, the Y-axis, and the Z-axis.

A subject 130 is placed on the top plate 15 of the bed 14. The receive coil 110 corresponding to an examination part is attached to the subject 130. The top plate 15 on which the subject 130 is placed is moved into the imaging space 18. Therefore, the examination part of the subject 130 is positioned at the center of the static magnetic field in the imaging space 18.

The RF transmission coil 106 is a coil that irradiates the subject 130 with an RF pulse which is a high-frequency magnetic field pulse. The RF transmission coil 106 is connected to the high-frequency magnetic field generator 116 and is supplied with a high-frequency pulse current from the high-frequency magnetic field generator 116.

The MRI apparatus 10 comprises a sequencer 118, a controller 120, and an operation unit 122. The sequencer 118 transmits commands to the high-frequency magnetic field generator 116 and the gradient magnetic field power supply 114 according to an imaging pulse sequence. Then, an appropriately amplified signal is transmitted to each of the RF transmission coil 106 and the gradient magnetic field coil 104.

The high-frequency magnetic field generator 116 is driven in response to a command from the sequencer 118 to modulate the amplitude of a high-frequency pulse and supplies the amplified high-frequency pulse current to the RF transmission coil 106. The RF transmission coil 106 applies, to the subject 130, a pulsed high-frequency magnetic field as an RF pulse corresponding to a signal from the high-frequency magnetic field generator 116. Preferably, the sequencer 118 sends a command to the receive coil 110 according to the imaging pulse sequence to turn off the receive coil 110 during a period for which the RF pulse is applied.

A nuclear magnetic resonance phenomenon is induced in a spin of an atom constituting a biological tissue of the subject 130 by the application of the high-frequency magnetic field. The receive coil 110 is a multi-channel RF coil unit including a plurality of coil elements for receiving a nuclear magnetic resonance signal generated in the subject 130. In addition, nuclear magnetic resonance may be referred to as NMR using an abbreviation of Nuclear Magnetic Resonance. Further, the nuclear magnetic resonance signal can be referred to as an NMR signal.

The NMR signal generated from the subject 130 is detected by the receive coil 110, is amplified by a preamplifier (not shown) in the receive coil 110, and is transmitted to the receiver 112. In the receiver 112, analog-to-digital conversion and necessary signal processing are performed on the amplified NMR signal to generate data. The generated data is transmitted to the controller 120. This data is referred to as a received signal or measurement data.

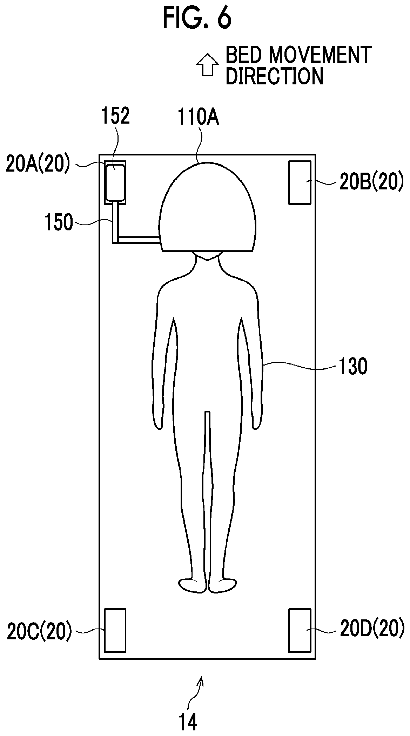

A reception-side cable for outputting the NMR signal received by the receive coil 110 is connected to the receive coil 110. A reception-side connector is connected to an end portion of the reception-side cable. The reception-side connector is connected to a bed-side connector of a bed-side cable. The bed-side cable is connected to the controller 120. Therefore, the receive coil 110, the controller 120, and the sequencer 118 are connected such that they can communicate with each other. Further, in Fig. 2, the reception-side cable, the reception-side connector, and the bed-side connector of the bed-side cable are not shown. The reception-side cable, the reception-side connector, and the bed-side connector of the bed-side cable are shown in Fig. 6 and the like.

The sequencer 118 controls the operation of each unit according to the timing and intensity programmed in advance. Among programs, a program that describes the timing and intensity of each of the RF pulse, the gradient magnetic field, and signal reception is referred to as the imaging pulse sequence. The imaging pulse sequence can be referred to as a pulse sequence. Various types of imaging pulse sequences are known depending on the purpose, but a detailed description thereof will be omitted here.

The controller 120 receives various inputs transmitted from the operation unit 122 and integrally controls each unit of the MRI apparatus 10 through the sequencer 118. In addition, the controller 120 performs, for example, a process of performing inverse Fourier transform on a received signal in a spatial frequency domain transmitted from the receiver 112 to convert the received signal into an image in a real space, thereby generating an MRI image.

The operation unit 122 includes input devices such as a mouse and a keyboard. The operation unit 122 includes a display device such as a liquid crystal display. The operation unit 122 functions as a user interface used by a user, such as a technician, to start, stop, or pause the MRI apparatus 10, to select a pulse sequence, and to input imaging conditions, processing conditions, and the like. Further, the user interface can be referred to as a UI using an abbreviation of User Interface.

The sequencer 118 and the controller 120 can be configured using a computer. In addition, a computer system including a plurality of computers may be applied as the processing functions of the sequencer 118 and the controller 120.

Furthermore, the MRI apparatus 10 shown in Figs. 1 and 2 is an example of a medical imaging apparatus comprising an imaging unit that generates imaging data of the subject in the present disclosure and an image reconstruction unit that generates a reconstruction based on the imaging data.

### Example of Hardware Configuration of Information Processing Apparatus

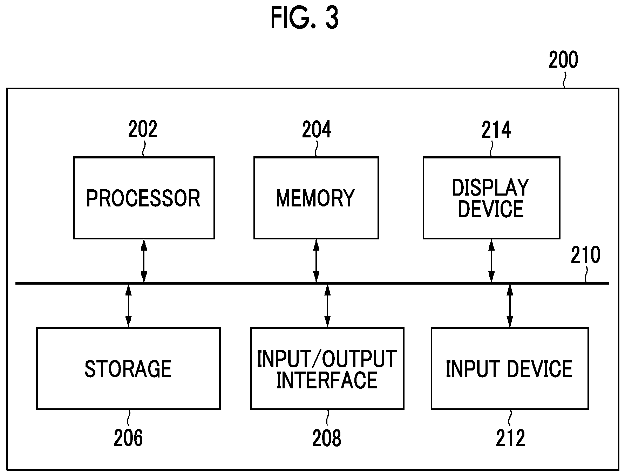

Fig. 3 is a block diagram showing an example of a hardware configuration of an information processing apparatus used in the MRI apparatus. An information processing apparatus 200 can function as the controller 120 and the operation unit 122 shown in Fig. 2. The information processing apparatus 200 may function as the sequencer 118.

The information processing apparatus 200 may be a personal computer, a workstation, or a server computer. The computer may be a virtual machine.

In the information processing apparatus 200, at least some of the functions may be implemented by cloud computing. At least some of the functions of the information processing apparatus 200 may be provided as SaaS. In addition, SasS is an abbreviation of Software as a Service.

The information processing apparatus 200 comprises a processor 202, a memory 204 which is a main storage device, a storage 206 which is an auxiliary storage device, an input/output interface 208, and a bus 210. The processor 202 is connected to the memory 204, the storage 206, the input/output interface 208, an input device 212, and a display device 214 via the bus 210.

The processor 202 executes commands stored in the memory 204. A hardware structure of the processor 202 is various processors described below. The various processors include, for example, a CPU which is a general-purpose processor executing software to function as various functional units, a GPU which is a processor specialized in image processing, a PLD, such as an FPGA, which is a processor whose circuit configuration can be changed after manufacture, and a dedicated electric circuit, such as an ASIC, which is a processor having a dedicated circuit configuration designed to execute a specific process.

In addition, CPU is an abbreviation of Central Processing Unit, and GPU is an abbreviation of Graphics Processing Unit. FPGA is an abbreviation of Field Programmable Gate Array, PLD is an abbreviation of Programmable Logic Device, and ASIC is an abbreviation of Application Specific Integrated Circuit. The software is synonymous with a program.

One processing unit may be configured by one of the various processors or by a combination of two or more processors of the same type or different types. Examples of the two or more processors include a plurality of FPGAs, a combination of a CPU and an FPGA, and a combination of a CPU and a GPU.

Further, a plurality of processing units may be configured by one processor. A first example in which a plurality of processing units are configured by one processor is an aspect in which one processor is configured by a combination of one or more CPUs and software and functions as a plurality of processing units. A representative example of this aspect is a client computer or a server computer.

A second example in which a plurality of processing units are configured by one processor is an aspect in which a processor that implements the functions of the entire system including a plurality of processing units using one IC chip is used. A representative example of this aspect is an SoC. As described above, various processing units are configured using one or more of the various processors as the hardware structure.

In addition, specifically, the hardware structure of the various processors is an electric circuit (circuitry) obtained by combining circuit elements such as semiconductor elements. Further, SoC is an abbreviation of System On Chip, and IC is an abbreviation of Integrated Circuit.

The memory 204 includes a RAM. The memory 204 may include a ROM. The storage 206 may be, for example, a hard disk drive, a solid state drive, or a plurality of combinations thereof. In addition, the storage 206 may include an external storage device such as a removable medium.

Furthermore, RAM is an abbreviation of Random Access Memory, and ROM is an abbreviation of Read Only Memory. The hard disk drive can be referred to as an HDD using an abbreviation of Hard Disk Drive. The solid state drive can be referred to as an SSD using an abbreviation of Solid State Drive.

The storage device including the memory 204 and the storage 206 stores programs, data, and the like for implementing various functions of the MRI apparatus 10. The processor 202 executes the programs stored in the memory 204 to implement various functions. The processor 202 integrally controls each unit of the information processing apparatus 200, various devices and units provided in the MRI apparatus 10, and the like and performs various processes.

The input/output interface 208 includes a communication interface that can be connected to a telecommunication line, such as a local area network, a connection interface that can be connected to an external device, and the like. For example, a universal serial bus, HDMI (HDMI is a registered trademark), or the like can be applied as the connection interface that can be connected to the external device. HDMI is an abbreviation of High-Definition Multimedia Interface.

The processor 202 communicates with various devices of the MRI apparatus 10 via the input/output interface 208 to transmit and receive various types of information.

Examples of the input device 212 include a keyboard and a pointing device such as a mouse. The input device 212 may include a numeric keypad, various switch buttons, and the like. The input device 212 may include a voice input device. The input device 212 may be a touch panel input device that is configured integrally with a display screen of the display device 214.

The display device 214 may be a liquid crystal display, an organic EL display, or a projector. The display device 214 may be an appropriate combination of a liquid crystal display and the like. The display device 214 displays various types of information, in addition to the image captured by the MRI apparatus 10. The display device 214 is used as a portion of a UI in a case where an input is received from the input device 212. The display device 214 is not limited to a single display device, and a form of a multi-display comprising a plurality of display devices can also be adopted. In addition, the organic EL may be referred to as OEL using an abbreviation of Organic Electro-Luminescence.

Further, the memory 204 shown in Fig. 3 is an example of a first memory according to the embodiment of the present disclosure.

### Overview of Procedure of MRI Imaging

### A typical imaging procedure applied to the MRI apparatus 10 is generally as follows.

### Procedure 1

An operator, such as a technician, sets subject information, an imaging part corresponding to a target disease, an examination protocol in which an imaging type and the like are defined, and other information necessary for an examination, using the input device 212 such as a mouse and a keyboard. The operator refers to a user and an operating person.

The subject information includes a name of the subject, a subject code, and the like. Some or all of these information items may be manually input by the operator through the input device 212, or information stored in advance in a recording medium or the like may be read. In addition, the operator finally checks parameters that are automatically set or input and manually sets the parameters through the input device 212 as necessary.

### Procedure 2

The subject 130 is placed on the top plate 15 of the bed 14, and the receive coil 110 is attached to the subject 130. The operator connects the reception-side connector of the receive coil 110 to the closest bed-side connector.

In a case where the receive coil 110 is connected to the bed-side connector, a body position of the subject 130 is determined. The body position of the subject 130 includes a posture of the subject 130 and an insertion direction of the subject 130 into the gantry 12.

Examples of the posture of the subject 130 include a supine posture, a prone posture, a right lateral decubitus posture, and a left lateral decubitus posture. For example, the posture of the subject 130 and the insertion direction of the subject 130 into the gantry 12 may be head-first or feet-first posture and direction.

### Procedure 3

The top plate 15 is moved to the imaging space 18 using a gantry operation panel, a foot switch, or the like, and an examination target part of the subject 130 is moved to a static magnetic field center position of the gantry 12. Further, in Figs. 1 and 2, the gantry operation panel and the foot switch are not shown.

### Procedure 4

A scanogram is captured, and a positioning image is acquired. In the capture of the scanogram, a plurality of slices are imaged for one or more of an axial plane, a coronal plane, and a sagittal plane. In the case of a cardiac MRI examination, imaging is performed for an imaging range covering a three-dimensional region including a heart of the subject 130 in the capture of the scanogram in synchronization with an electrocardiogram waveform to acquire a 3D scanogram image. In addition, a slice thickness in the capture of the scanogram may be set to a value larger than a slice thickness in main imaging.

### Procedure 5

After the capture of the scanogram is ended, the positioning image is reconstructed. A reconstructed 3D scanogram image is displayed on the display device 214 of the operation unit 122.

### Procedure 6

The operator sets an imaging position of the main imaging based on a cross-sectional image generated from the 3D scanogram image. In the MRI apparatus 10 according to the present embodiment, an imaging position including a slice position, which is a cross section position to be measured in the main imaging, is automatically calculated from the 3D scanogram image, and a recommended imaging position is presented to the operator. The operator checks the presented slice position and the like and manually adjusts the imaging position using the input device 212 of the operation unit 122 as necessary.

Further, the operator sets imaging parameters to be applied to the main imaging. Some or all of the imaging parameters may be input by the user, such as the technician, through the operation unit 122 or may be automatically set or input. The operator finally checks the automatically set (automatically input) parameters and manually sets the parameters using the input device 212 of the operation unit 122 as necessary.

### Procedure 7

The main imaging is executed according to the imaging parameters and the imaging position set in procedure 6.

### Procedure 8

The main imaging is executed, and the image is reconstructed. The reconstructed image is displayed on the display device 214 of the operation unit 122.

### Procedure 9

The operator sets a window value of a value suitable for diagnosis for the reconstructed image displayed on the display device 214, using the input device 212 of the operation unit 122, and obtains an image to be used for diagnosis.

### Procedure 10

In a case where the imaging is completed, the top plate 15 is exited from the imaging space 18, and the subject 130 is exited from the gantry 12. The subject 130 comes down from the bed 14, and the MRI examination is ended.

### Procedure of Imaging Support Method According to Embodiment

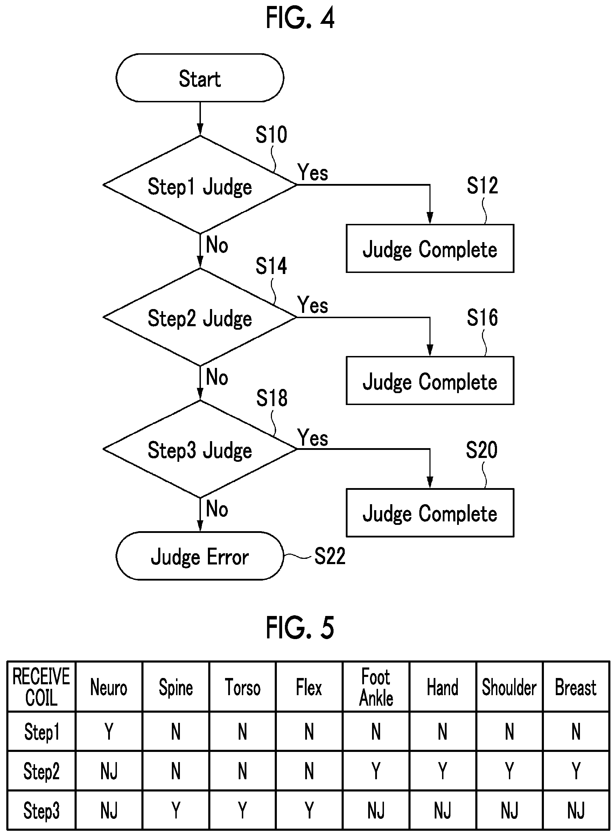

Fig. 4 is a flowchart showing an imaging support method according to the embodiment. Fig. 4 shows a procedure applied to the determination of the body position of the subject 130 before the subject 130 shown in Fig. 2 is moved into the imaging space 18. The processor 202 provided in the information processing apparatus 200 shown in Fig. 3 reads out a program from the memory 204 and executes commands included in the program to implement the determination of the body position of the subject 130. In addition, the imaging support method whose procedure is shown in Fig. 4 is an example of a method for operating a medical imaging support device.

In Step S10, receive coil information indicating the type of the receive coil 110 is acquired, and the body position of the subject 130 placed on the bed 14 is determined according to the type of the receive coil 110 connected to the bed 14.

In a case where the body position of the subject 130 is determined in Step S10, the determination result is Yes. In a case where the determination result is Yes, the process proceeds to Step S12. In Step S12, the determination result is stored. In Step S12, information indicating the body position of the subject 130 may be displayed on the display device 214.

On the other hand, in a case where the body position of the subject 130 is not determined in Step S10, the determination result is No. In a case where the determination result is No, the process proceeds to Step S14. In Step S14, receive coil connection position information indicating a connection position of the receive coil 110 in the bed 14 is acquired. In addition, Step S10 is an example of a first step according to the present disclosure.

In Step S14, the body position of the subject 130 placed on the bed 14 is determined according to the receive coil connection position information, in addition to the receive coil information acquired in Step S10.

The cable of the receive coil 110 is designed to have a minimum length in order to reduce noise. The reception-side connector of the receive coil 110 is connected to the closest bed-side connector.

In a case where the body position of the subject 130 is determined in Step S14, the determination result is Yes. In a case where the determination result is Yes, the process proceeds to Step S16. In Step S16, the determination result is stored. In Step S14, the information indicating the body position of the subject 130 may be displayed on the display device 214.

On the other hand, in a case where the body position of the subject 130 is not determined in Step S14, the determination result is No. In a case where the determination result is No, the process proceeds to Step S18. In Step S18, at least one of a result of image recognition on the physical features of the subject 130 or a result of image recognition on the structural features of the receive coil 110 is acquired. In addition, Step S14 is an example of a second step according to the present disclosure.

In Step S18, at least one of the physical features of the subject 130 or the structural features of the receive coil 110 is recognized from the captured image of the subject 130. In Step S18, the body position of the subject 130 placed on the bed 14 is determined according to the result of the image recognition of the subject 130, in addition to the receive coil information and the receive coil connection position information.

In the image recognition of the subject 130, in a case where neither the physical features of the subject 130 nor the structural features of the receive coil 110 can be recognized, face recognition of the subject 130 is performed, and the body position of the subject 130 is determined based on a result of the face recognition of the subject 130.

In a case where the body position of the subject 130 is determined in Step S18, the determination result is Yes. In a case where the determination result is Yes, the process proceeds to Step S20. In Step S20, the determination result is stored. In Step S20, the information indicating the body position of the subject 130 may be displayed on the display device 214.

On the other hand, in a case where the body position of the subject 130 is not determined in Step S18, the determination result is No. In a case where the determination result is No, the process proceeds to Step S22. In Step S22, error processing in the determination of the body position of the subject 130 is executed.

In Step S22, an error indicating that it is difficult to determine the body position of the subject 130 may be notified. Examples of the notification of the error include an aspect in which a character expression indicating the error is displayed using the display device 214 and an aspect in which sound information, such as an alarm sound, indicating the error is applied.

In Step S18, the determination of the body position of the subject 130 based on the physical features of the subject 130, the determination of the body position of the subject 130 based on the structural features of the receive coil 110, and the determination of the body position of the subject 130 based on the face recognition of the subject 130 may be executed as different steps.

Further, Step S18 is an example of a third step according to the present disclosure. Each of the physical features of the subject 130 obtained by analyzing the captured image of the subject and the result of the face recognition of the subject 130 is an example of subject feature information according to the present disclosure. The structural features of the receive coil 110 are an example of receive coil feature information generated based on the analysis result of the captured image of the receive coil attached to the subject.

### Specific Example of Relationship Between Type of Receive Coil and Step of Determining Body Position

Fig. 5 is a table showing a correspondence relationship between the type of the receive coil and the step of determining the body position of the subject. The correspondence relationship between the type of the receive coil 110 and the step of determining the body position of the subject 130 shown in Fig. 5 is stored in a coil-body position correspondence information storage unit 221 shown in Fig. 13.

In Fig. 5, a neuro coil, a spine coil, a flex coil, a torso coil, a foot ankle coil, a hand coil, a shoulder coil, and a breast coil are shown as the types of the receive coils. However, some of the receive coils 110 shown in Fig. 5 may be stored, and other receive coils 110 may be added. An identifier given for each type of receive coil may be applied as the type of the receive coil.

Step 1, Step 2, and Step 3 shown in Fig. 5 correspond to Step S10, Step S14, and Step S18 shown in Fig. 4, respectively. Y in Fig. 5 represents a "Yes" determination in each step. N in Fig. 5 represents a "No" determination in each step. NJ in Fig. 5 represents that the process does not proceed to the next step and the determination is not performed in each of the next steps.

For example, in a case where the receive coil information indicating that the neuro coil attached to the head of the subject 130 is connected to the bed 14 is acquired, the body position of the subject 130 is determined based on the receive coil information in Step 1.

In a case where the receive coil information indicating that the foot ankle coil attached to the foot or the like of the subject 130 is connected to the bed 14 is acquired, the body position of the subject 130 is determined based on the receive coil information and the receive coil connection position information in Step 2.

In a case where the receive coil information indicating that the torso coil, which is a general-purpose receive coil that can be used for a plurality of examination parts, is connected to the bed 14 is acquired, the body position of the subject 130 is determined based on the receive coil information, the receive coil connection position information, and the physical features of the subject 130 in Step 3.

In a case where the subject 130 is covered with a blanket such that it is difficult to recognize the physical features of the subject 130 from a captured image obtained by imaging the whole body of the subject 130, the body position of the subject 130 is determined based on the result of the face recognition on the captured image of the subject 130.

In a case where the receive coil 110 for determining the body position of the subject 130 in Step 1 and Step 2 and for determining the body position of the subject 130 without performing Step 3 as the step of determining the body position of the subject 130 is used, a correspondence relationship that does not include Step 3 may be stored.

### Specific Example of Determination of Body Position

### Case Where Neuro Coil Is Used

Fig. 6 is a schematic view showing the subject for which the neuro coil is used. Fig. 6 is a schematic view showing the bed 14 on which the subject 130 is placed as viewed from the side of the surface on which the subject 130 is placed.

The bed 14 includes a plurality of bed-side connectors 20. In the bed 14 shown in Fig. 6, a bed-side connector 20A and a bed-side connector 20B are disposed in one end portion in a longitudinal direction, and a bed-side connector 20C and a bed-side connector 20D are disposed in the other end portion in the longitudinal direction. Fig. 6 shows an example in which a side closer to the gantry 12 in a bed movement direction, which is a movement direction of the bed 14, is one end of the bed 14 in the longitudinal direction, and a side opposite to the gantry 12 in the bed movement direction is the other end of the bed 14 in the longitudinal direction.

The bed-side connector 20A and the bed-side connector 20C are disposed in one end portion of the bed 14 in a lateral direction. The bed-side connector 20B and the bed-side connector 20D are disposed in the other end portion of the bed 14 in the lateral direction.

The bed-side connector 20A, the bed-side connector 20B, the bed-side connector 20C, and the bed-side connector 20D have the same shape, and a reception-side connector 152 of the receive coil 110 is connected to each of the bed-side connectors. Fig. 6 shows a state in which a receive coil 110A, which is the neuro coil, is connected to the bed-side connector 20A through a reception-side cable 150 and the reception-side connector 152.

The reception-side cable 150 is an example of an electric wire according to the present disclosure. The reception-side connector 152 is an example of a first terminal according to the present disclosure.

Fig. 6 shows an aspect in which the bed-side connector 20 is disposed at each of four corners of the bed 14. However, the number of bed-side connectors and the disposition thereof are not limited to the aspect shown in Fig. 6. For example, the bed-side connectors may be disposed in a central portion of the bed 14 in the longitudinal direction and in a central portion of the bed 14 in the lateral direction. In addition, the bed-side connector 20 is an example of a second terminal according to the present disclosure.

Here, the end portion of the bed 14 represents a prescribed range from the end which includes the end of the bed 14. The center portion of the bed 14 represents a prescribed range from the center of the bed 14 which includes the center of the bed 14.

The neuro coil shown in Fig. 6 is a dedicated specification for the supine posture as a receive coil for the head and is fixed at a position of the bed 14 closer to the gantry 12. In a case where the neuro coil is connected to the bed-side connector 20A, it is determined that the insertion direction of the subject 130 into the imaging space 18 is the head-first direction and the subject 130 is in the supine posture. In a case where the receive coil information acquired in Step S10 indicates the neuro coil, it is possible to determine that the body position of the subject 130 is the head-first position and is the supine posture, using the receive coil information. The same applies to a case where the neuro coil is connected to the bed-side connector 20B.

### Case Where Foot Ankle Coil Is Used

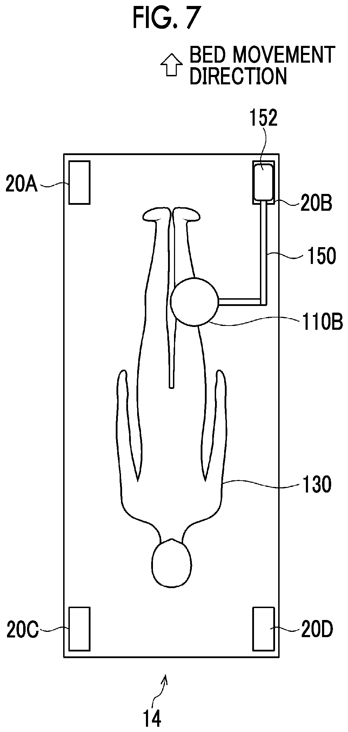

Fig. 7 is a schematic view showing the subject for which the foot ankle coil is used. Fig. 7 shows a state in which a receive coil 110B, which is the foot ankle coil, is connected to the bed-side connector 20B. The foot ankle coil is applied to the supine posture. However, it is not known whether the insertion direction of the subject 130 into the imaging space 18 is the head-first direction or the feet-first direction.

That is, in a case where the receive coil information acquired in Step S10 indicates the foot ankle coil, the process proceeds to Step S14 in Fig. 5, and it is determined whether the insertion direction of the subject 130 into the imaging space 18 is the head-first direction or the feet-first direction, using the receive coil connection position information.

In Fig. 7, it is determined that the body position of the subject 130 is the supine posture and is the feet-first position based on the fact that the reception-side connector 152 of the receive coil 110B, which is the foot ankle coil, is connected to the bed-side connector 20B. The same applies to a case where the receive coil 110B is connected to the bed-side connector 20A.

### Case Where Torso Coil Is Used

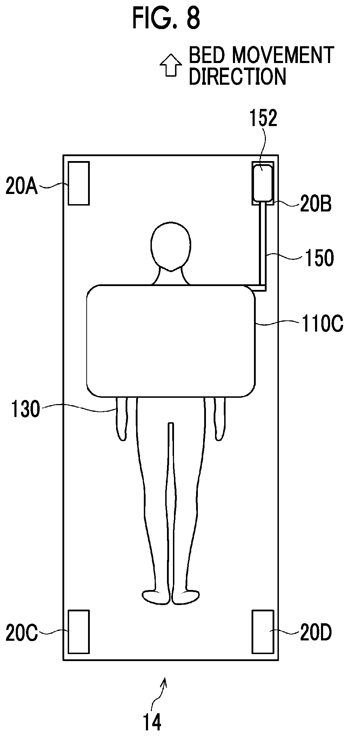

Fig. 8 is a schematic view showing the subject for which the torso coil is used. Fig. 8 shows a state in which a receive coil 110C, which is the torso coil, is connected to the bed-side connector 20B.

The torso coil is a general-purpose receive coil that can be used for a plurality of different parts. In addition, the torso coil has a larger size than other receive coils and covers the body of the subject 130. In this case, in a case where the torso coil is used, it is difficult to determine the body position of the subject 130 based on the receive coil information and the receive coil connection position information.

In a case where the torso coil is used and the physical features of the subject 130 are recognized from the image recognition of the subject 130, the body position of the subject 130 is determined based on the physical features of the subject 130.

On the other hand, in a case where the torso coil is used and it is difficult to recognize the physical features of the subject 130, the structural features of the torso coil are recognized, the shape, size, and the like of a body part of the subject 130 protruding from the torso coil are recognized, and the body position of the subject 130 is determined based on the recognition result.

In a case where the receive coil information acquired in Step S10 indicates the torso coil, the process proceeds to Step S14, and the receive coil connection position information is acquired. Further, the process proceeds to Step S18, and information of the physical features of the subject 130 and information of the structural features of the receive coil 110C are acquired.

In Step S18, the receive coil information and the receive coil connection position information are used, and the body position of the subject 130 is determined based on at least one of the physical features derived from the image recognition of the subject 130 or the structural features of the receive coil 110. In Step S18, the body position of the subject 130 may be determined from the result of the image recognition of the subject 130, without using the receive coil information and the receive coil connection position information.

For example, in the example shown in Fig. 8, in a case where the face is recognized in the captured image of the subject 130, it is determined that the body position is the supine posture. In addition, in a case where a camera that images the subject 130 is disposed in the gantry 12 and the head of the subject 130 is recognized on the front side of a captured image of the subject 130, it is determined that the body position is the head-first position.

On the other hand, in a case where the head is recognized in the captured image of the subject 130, but the face is not recognized, it is determined that the body position is the prone posture. In addition, in a case where the camera that images the subject 130 is disposed in the gantry 12 and the foot of the subject 130 is recognized on the front side of the captured image of the subject 130, it is determined that the body position is the feet-first position.

### Case Where Blanket Is Used

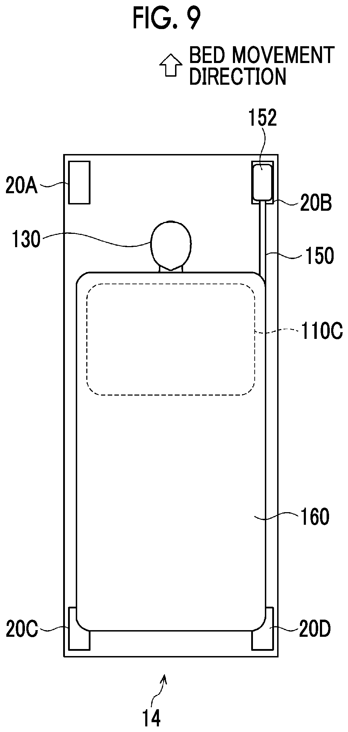

Fig. 9 is a schematic view showing the subject for which a blanket is used. Fig. 9 shows a state in which parts other than the head of the subject 130 are covered with a blanket 160 in a case where the receive coil 110C shown in Fig. 8 is used.

In a case where the subject 130 is covered with the blanket, in the image recognition on the image of the subject 130 captured by the camera, it is difficult to recognize the physical features of the subject 130. Therefore, the face recognition of the subject 130 is performed, and the body position of the subject 130 is determined.

In the example shown in Fig. 9, in a case where the face is recognized in the captured image of the subject 130, it is determined that the body position is the supine posture. In addition, in a case where the camera that images the subject 130 is disposed in the gantry 12 and the face of the subject 130 is recognized on the front side of the captured image of the subject 130, it is determined that the body position is the head-first position.

On the other hand, in a case where the head is recognized in the captured image of the subject 130, but the face is not recognized, it is determined that the body position is the prone posture. In addition, in a case where the camera that images the subject 130 is disposed in the gantry 12 and the head of the subject 130 is not recognized on the front side of the captured image of the subject 130, it is determined that the body position is the feet-first position.

### Case Where Breast Coil Is Used

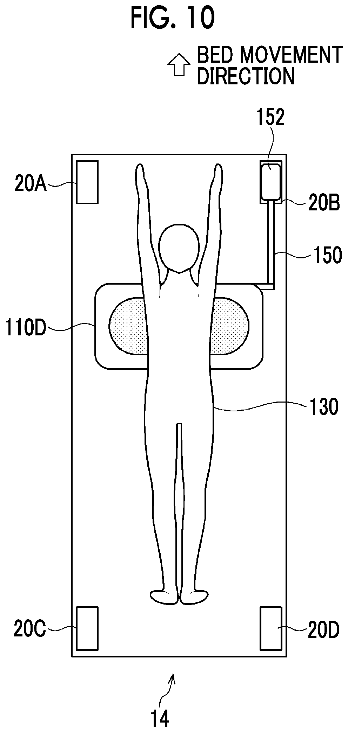

Fig. 10 is a schematic view showing the subject for which the breast coil is used. Fig. 10 shows a state in which the receive coil 110D, which is the breast coil, is connected to the bed-side connector 20B.

The breast coil is a receive coil for the breast and is used only in the prone posture. In a case where the receive coil information indicating that the breast coil is applied is acquired in Step S10 of Fig. 4, it is determined in Step S10 that the body position is the prone posture. Further, the breast coil can be applied to either the head-first position or the feet-first position, the body position of the subject 130 is not determined in Step S10, and the process proceeds to Step S14.

In Step S14, in a case where the receive coil connection position information indicating that the reception-side connector 152 is connected to the bed-side connector 20B is acquired, the body position is determined to be the head-first position, and it is determined that the body position of the subject 130 is the prone posture and is the head-first position.

In Step S14, even in a case where the receive coil connection position information indicating that the reception-side connector 152 is connected to the bed-side connector 20A is acquired, it is determined that the body position is the head-first position.

On the other hand, in Step S14, even in a case where the receive coil connection position information indicating that the reception-side connector 152 is connected to the bed-side connector 20C or the bed-side connector 20D is acquired, it is determined that the body position is the feet-first position.

### Specific Example of Determination of Body Position Based on Face Recognition

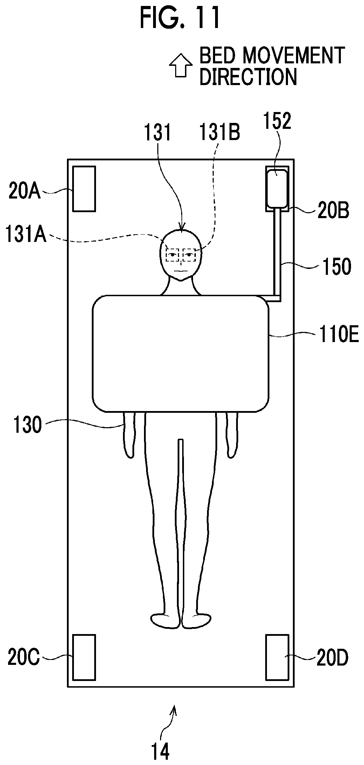

Fig. 11 is a schematic view showing the subject in a case where both eyes are recognized in the face recognition of the subject. Fig. 11 schematically shows a case where a receive coil 110E that makes it difficult to determine the supine posture of the subject 130 based on the receive coil information is used. Examples of the receive coil 110E include the spine coil attached to the back of the subject 130 and the flex coil attached to be wound around the examination part, which are shown in Fig. 5.

Fig. 11 shows a case where a face 131 is recognized in the head of the subject 130 and both a right eye 131A and a left eye 131B of the subject 130 are recognized. In a case where both eyes of the subject 130 are recognized, it is determined that the body position is the supine posture in Step S18 of Fig. 4.

In a case where the face recognition of the subject 130 is performed and neither the right eye 131A nor the left eye 131B of the subject 130 is recognized, it is determined that the body position is the prone posture in Step S18 of Fig. 4. In addition, a case where neither eye is recognized in Step S18 is not shown.

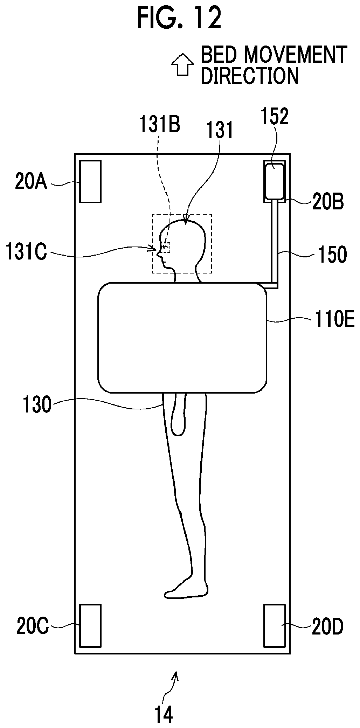

Fig. 12 is a schematic view showing the subject in a case where one eye is recognized in the face recognition of the subject. Fig. 12 schematically shows a case where the receive coil 110E that makes it difficult to determine the supine posture of the subject 130 based on the receive coil information is used, similarly to Fig. 11.

Fig. 12 shows a case where the face 131 is recognized in the head of the subject 130 and the left eye 131B of the subject 130 is recognized. In a case where only the left eye 131B or only the right eye 131A of the subject 130 is recognized, it is determined that the body position is the lateral decubitus posture in Step S18 of Fig. 4.

Further, it may be determined whether the body position is a left decubitus posture or a right decubitus posture according to the position of the eye with respect to the entire head or the position of a nose 131C with respect to the entire head. Fig. 12 shows an example of the left decubitus posture.

In the example shown in Figs. 11 and 12, it may be determined whether the body position is the head-first position or the feet-first position, based on the position of the camera that images the subject 130 and the position of the face in the captured image of the subject 130.

For example, in a case where the camera that images the subject 130 is disposed at a position of the gantry 12 and the face 131 of the subject 130 is recognized at a position on the front side of the receive coil 110 in the captured image of the subject 130, it may be determined that the body position is the head-first position. On the other hand, in a case where the face 131 of the subject 130 is recognized at a position on the back side of the receive coil 110 in the captured image of the subject 130, it may be determined that the body position is the feet-first position.

### Example of Configuration of Imaging Support Device According to Embodiment

Fig. 13 is a functional block diagram showing an electric configuration of an imaging support device according to the embodiment. An imaging support device 220 shown in Fig. 13 is provided in the information processing apparatus 200 provided in the MRI apparatus 10.

The imaging support device 220 comprises the coil-body position correspondence information storage unit 221. The coil-body position correspondence information storage unit 221 stores a correspondence relationship between the type of the receive coil and the step of determining the body position of the subject shown in Fig. 5.

The imaging support device 220 comprises a receive coil information acquisition unit 222. The receive coil information acquisition unit 222 acquires the receive coil information indicating the type of the receive coil 110. An identifier that is defined for each type of the receive coil 110 and is given to the receive coil 110 may be applied as the receive coil information. The receive coil information acquisition unit 222 stores the acquired receive coil information in a prescribed memory.

The receive coil information acquisition unit 222 may acquire the receive coil information in a case where the reception-side connector 152 of the receive coil 110 is connected to the bed-side connector 20. The receive coil information acquisition unit 222 may acquire the receive coil information input by the operator through the operation unit 122.

The imaging support device 220 comprises a receive coil connection information acquisition unit 224. The receive coil connection information acquisition unit 224 acquires information of the position of the bed-side connector 20 to which the receive coil 110 is connected. That is, in a case where the reception-side connector 152 of the receive coil 110 is connected to any of the plurality of bed-side connectors 20, it is recognized to which of the plurality of bed-side connectors 20 the reception-side connector 152 is connected.

The receive coil information acquisition unit 222 and the receive coil connection information acquisition unit 224 acquire information indicating which type of receive coil 110 is connected to which position of the bed-side connector 20 and store the acquired information in the prescribed memory. Further, in Fig. 13, the memory in which the receive coil information and the receive coil connection information are stored is not shown. The receive coil information and the receive coil connection information may be stored in the memory 204 shown in Fig. 3.

The imaging support device 220 comprises a sensor data acquisition unit 226. The sensor data acquisition unit 226 acquires sensor data for the detection of the subject 130 from the sensor 228 provided outside the imaging support device 220.

That is, the imaging support device 220 acquires the information of the subject 130 placed on the bed 14 and the information of the receive coil 110 connected to the bed 14 in cooperation with an external information input device such as the sensor 228. Examples of the sensor 228 that functions as the external information input device include an optical camera, an infrared camera, and a millimeter-wave camera.

The imaging support device 220 comprises a sensor data processing unit 230. The sensor data processing unit 230 performs prescribed signal processing on the sensor data acquired by the sensor data acquisition unit 226. The function of the sensor data processing unit 230 may be comprised in the sensor 228 or the sensor data acquisition unit 226.

For example, in a case where the optical camera is applied as the sensor 228, the sensor data processing unit 230 can generate information indicating the physical features of the subject 130 from a captured image obtained by imaging the whole body of the subject 130 to which the receive coil 110 is attached. The sensor data processing unit 230 can generate information indicating the structural features of the receive coil 110 from the captured image of the subject 130. The sensor data processing unit 230 can recognize the face from the captured image of the subject 130.

The imaging support device 220 comprises a body position determination unit 232. The body position determination unit 232 determines the body position of the subject 130 based on at least one of the receive coil information, the receive coil connection information, the physical features of the subject 130, or the structural features of the receive coil 110. The body position of the subject 130 includes the supine posture of the subject 130 and the insertion direction of the subject 130 into the imaging space 18.

The imaging support device 220 comprises a display signal generation unit 234. The display signal generation unit 234 generates a display signal that indicates subject body position information indicating the body position of the subject 130 determined by the body position determination unit 232. The display signal generation unit 234 transmits the display signal to the display device 214. The display device 214 displays the subject body position information.

In addition, the imaging support device 220 shown in Fig. 13 is an example of a medical imaging support device according to the present disclosure. The coil-body position correspondence information storage unit 221 is an example of a second memory according to the present disclosure.

### Specific Example of Rewriting of Correspondence Relationship

Hereinafter, the correspondence relationship between the type of the receive coil and the step of determining the body position of the subject shown in Fig. 5 is simply referred to as a correspondence relationship. The correspondence relationship shown in Fig. 5 can be rewritten by the operator.

For example, in a case where the receive coil 110 that is not stored as the correspondence relationship is added, the correspondence relationship that has already been stored can be rewritten to determine the body position of the subject 130 corresponding to the newly added receive coil 110.

In particular, this configuration is characterized by a method of rewriting the correspondence relationship, and the identifier of the receive coil 110 is applied as the receive coil information, which makes it possible to ascertain whether or not the determination of the body position of the subject 130 is completed in Step 1 of Fig. 5, whether or not the process needs to be performed up to Step 2, or whether or not the process needs to be performed up to Step 3 in the imaging support device 220.

Specifically, the identifier of the receive coil 110 may include information of the steps necessary for the determination of the body position of the subject 130. In addition, the information of the newly added receive coil 110 may be acquired from a remote service connected to the imaging support device 220 for maintenance.

The correspondence relationship may be automatically updated. In a case where the correspondence relationship is updated, information used in other facilities may be taken into account. The other facilities are other hospitals, other examination institutions, and the like in which the MRI apparatus 10 provided with the imaging support device 220 is used.

Specifically, the imaging support device 220 provided in each facility stores the body position of the subject 130 determined by the imaging support device 220 in each step and the actual body position of the subject 130 for the remote service connected for maintenance. In the remote service, in a case where it is found that the body position of the subject 130 can be determined with the number of steps smaller than the number of steps already defined and the correspondence relationship in each facility is updated, the number of steps already defined is updated to a smaller number of steps.

The correspondence relationship may be automatically learned based on the features of each facility. Specifically, a system may be adopted in which the body position of the subject 130 that is used frequently is defined for each facility and the body position of the subject 130 that is used frequently is preferentially determined.

The correspondence relationship may be prepared for each operator and may be selectively switched according to operator information. Specifically, a system may be adopted in which the body position of the subject 130 that is used frequently is defined for each operator and the body position of the subject 130 that is used frequently is preferentially determined.

That is, the imaging support device 220 may comprise an operator information acquisition unit that acquires operator information, such as a name of the operator and an identification number of the operator, and the body position determination unit 232 may determine the body position of the subject 130 using a correspondence relationship corresponding to the operator information.

### Operation and Effect of Embodiment

The imaging support device and the imaging support method according to the embodiment can obtain the following operation and effect.
[1] It is determined whether or not a plurality of steps of detecting the body position of the subject 130 need to be performed, with reference to the correspondence relationship between the type of the receive coil and the plurality of steps executed to detect the body position of the subject 130, based on the receive coil information indicating the type of the receive coil. Therefore, in the step suitable for the type of the receive coil, the body position of the subject 130 is determined, and the accurate and rapid recognition of the body position of the subject 130 is achieved. In addition, the operation of another device, such as a device for achieving high-precision image recognition, is suppressed.
[2] The plurality of steps include Step 1 of determining the body position of the subject 130 based on the type of the receive coil 110 and Step 2 of determining the body position of the subject 130 based on the type of the receive coil 110 and the bed-side connector 20 to which the receive coil 110 is connected. Therefore, in a case where it is difficult to determine whether the body position is the head-first position or the feet-first position from the type of the receive coil 110, it is possible to determine whether the body position is the head-first position or the feet-first position based on the bed-side connector 20 to which the receive coil 110 is connected.
[3] The reception-side connectors 152 connected to the receive coil 110 are disposed in an end portion of the bed 14 closer to the gantry 12 and an end portion of the bed 14 opposite to the gantry 12. Therefore, it is possible to determine whether the body position is the head-first position or the feet-first position based on a combination of the part for which the receive coil 110 is used and the position of the bed-side connector to which the reception-side connector 152 is connected.
[4] The correspondence relationship includes Step 3 of determining the body position of the subject 130 based on at least one of the physical features of the subject 130 or the structural features of the receive coil 110. In a case where the body position of the subject 130 is not determined in Step 1 and Step 2, Step 3 is executed. Therefore, even in a case where it is difficult to determine the body position of the subject 130 based on the receive coil information and the receive coil connection position information, it is possible to determine the body position of the subject 130.
[5] The physical features of the subject 130 include the result of face recognition on the captured image of the subject 130. Therefore, even in a case where parts other than the head of the subject 130 are covered with the blanket such that it is not possible to recognize the whole body of the subject 130, it is possible to determine the body position of the subject 130.
[6] In the face recognition of the subject 130, in a case where both the right eye 131A and the left eye 131B are recognized, it is determined that the body position is the supine posture. In a case where both eyes are not recognized, it is determined that the body position is the prone posture. In the face recognition of the subject 130, in a case where the right eye 131A or the left eye 131B is recognized, it is determined that the body position is the decubitus posture. Therefore, it is possible to determine the posture of the subject 130 based on the result of the face recognition of the subject 130.
[7] The structural features of the receive coil 110 are understood based on the analysis result of the captured image of the receive coil 110 attached to the subject 130. Therefore, even in a case where the size of the receive coil 110 is larger than that of the part of the subject 130 to which the receive coil 110 is attached such that the receive coil 110 covers the part to which the receive coil 110 is attached, it is possible to determine the body position of the subject 130 based on the size, shape, and the like of the subject 130 protruding from the receive coil 110.
[8] In a case where a new receive coil 110 that is not stored in the correspondence relationship is used, the update of the correspondence relationship to add the new receive coil 110 and the step applied to the determination of the body position of the subject 130 is performed. Therefore, it is possible to customize the correspondence relationship according to a use history of the receive coil 110.
[9] The correspondence relationship is updated based on information of the step of determining the body position of the subject 130 in the facility in which the MRI apparatus 10 is provided. Therefore, for example, it is possible to customize the correspondence relationship according to the operation of each facility such as a facility that determines the body position of the subject 130 without using Step 3.
[10] The correspondence relationship for each operator is prepared and is selected according to the operator information. Therefore, it is possible to customize the correspondence relationship according to the usage aspect for each operator.

### Examples of Application to Program and Program Product

The imaging support method according to the embodiment may be configured as a program or a program product that causes a processor or a computer including the processor to implement the functions of each step.

For example, a program or a program product may be configured to cause a computer to implement a function of acquiring the receive coil information, a function of determining whether or not to execute Steps 1 to 3 according to the receive coil information, and a function of executing a step determined to need to be executed.

The program or the program product may be stored in a computer-readable medium which is a non-transitory tangible information storage medium or may be provided through an information storage medium.

The present disclosure is not limited to the above-described embodiments, and various modifications can be made without departing from the gist of the technical idea of the present disclosure.

### Explanation of References

10: MRI apparatus
12: gantry
14: bed
15: top plate
16: leg portion
18: imaging space
20: bed-side connector
20A: bed-side connector
20B: bed-side connector
20C: bed-side connector
20D: bed-side connector
102: static magnetic field generating magnet
104: gradient magnetic field coil
106: RF transmission coil
110: receive coil
110A: receive coil
110B: receive coil
110C: receive coil
110D: receive coil
110E: receive coil
112: receiver
114: gradient magnetic field power supply
116: high-frequency magnetic field generator
118: sequencer
120: controller
122: operation unit
130: subject
131: face
131A: right eye
131B: left eye
131C: nose
150: reception-side cable
152: reception-side connector
160: blanket
200: information processing apparatus
202: processor
204: memory
206: storage
208: input/output interface
210: bus
212: input device
214: display device
220: imaging support device
221: coil-body position correspondence information storage unit
222: receive coil information acquisition unit
224: receive coil connection information acquisition unit
226: sensor data acquisition unit
228: sensor
230: sensor data processing unit
232: body position determination unit
234: display signal generation unit
S10 to S22: each step of imaging support method

## Claims

1. A medical imaging support device comprising:
a processor;
a first memory that stores a program to be executed by the processor; and
a second memory that stores a correspondence relationship between a type of a receive coil and a plurality of steps of determining a body position of a subject,
wherein the processor is configured to:
acquire receive coil information indicating a type of a receive coil used to capture a medical image of the subject;
determine whether or not the plurality of steps need to be performed according to the receive coil information; and
perform a step determined to need to be performed.

2. The medical imaging support device according to claim 1,
wherein the second memory stores, as the plurality of steps, a first step of determining the body position of the subject according to the type of the receive coil and a second step of determining the body position of the subject according to a connection position of the receive coil in a bed, and
the processor is configured to perform the second step in a case where the body position of the subject is not determined in the first step.

3. The medical imaging support device according to claim 2,
wherein the processor is configured to:
acquire receive coil connection position information indicating to which of a plurality of second terminals disposed at different positions of the bed a first terminal provided in an electric wire of the receive coil is connected; and
perform the second step using the receive coil connection position information.

4. The medical imaging support device according to claim 2 or 3,
wherein the second memory stores, as the plurality of steps, a third step of determining the body position of the subject based on at least one of subject feature information indicating a feature of the subject or receive coil feature information indicating a structural feature of the receive coil attached to the subject, and
the processor is configured to:
acquire at least one of the subject feature information or the receive coil feature information; and
perform the third step in a case where the body position of the subject is not determined in the second step.

5. The medical imaging support device according to claim 4,
wherein the subject feature information includes a physical feature of the subject obtained by analyzing a captured image of the subject.

6. The medical imaging support device according to claim 4,
wherein the subject feature information includes a result of face recognition on a captured image of the subject.

7. The medical imaging support device according to claim 6,
wherein the processor is configured to determine a posture of the subject placed on the bed based on the result of the face recognition in the third step.

8. The medical imaging support device according to any one of claims 4 to 7,
wherein the receive coil feature information is generated based on an analysis result of a captured image of the receive coil attached to the subject.

9. The medical imaging support device according to any one of claims 1 to 8,
wherein the processor is configured to perform update of the correspondence relationship to add a type of a receive coil that is not stored in the second memory.

10. The medical imaging support device according to any one of claims 1 to 9,
wherein the processor is configured to update the correspondence relationship based on information of a step of determining the body position of the subject used in a facility in which a medical imaging apparatus is provided.

11. The medical imaging support device according to any one of claims 1 to 10,
wherein the second memory stores the correspondence relationship for each operator, and
the processor is configured to:
acquire operator information; and
refer to the correspondence relationship based on the operator information.

12. A medical imaging apparatus comprising:
an imaging unit that images a subject and generates imaging data of the subject;
an image reconstruction unit that generates a reconstruction based on the imaging data; and
the medical imaging support device according to any one of claims 1 to 11.

13. A method for operating a medical imaging support device, the method being executed by a computer that includes a second memory storing a correspondence relationship between a type of a receive coil and a plurality of steps of determining a body position of a subject and that functions as the medical imaging support device, the method comprising:
acquiring by the computer, receive coil information indicating a type of a receive coil used to capture a medical image of the subject;
determining by the computer, whether or not the plurality of steps need to be performed according to the receive coil information; and
performing by the computer, a step determined to need to be performed.

14. The method for operating a medical imaging support device, according to claim 13,
wherein the second memory stores, as the plurality of steps, a first step of determining the body position of the subject according to the type of the receive coil and a second step of determining the body position of the subject according to a connection position of the receive coil in a bed, and
the method further comprises:
performing by the computer, the second step in a case where the body position of the subject is not determined in the first step.

15. A non-transitory, computer readable recording medium which records thereon a program causing for a computer to execute the method for operating a medical imaging support device, according to claim 13 or 14.
